# FASCICULE DE BREVET EUROPEEN

(11) **EP 3 212 164 B1**
(45) Date de publication et mention de la délivrance du brevet: **18.05.2022**
(21) Numéro de dépôt: 15797140.9
(22) Date de dépôt: 30.10.2015
(51) Int. Cl.: A61K 9/00, A61K 9/20, A61K 9/16, A61K 31/496, A61P 25/24

(54) **GRANULES DE PRINCIPE ACTIF A DOUBLE MASQUAGE DE GOUT, LEUR PROCEDE DE PREPARATION ET COMPRIMES ORODISPERSIBLES LES CONTENANT**
GRANULAT MIT DOPPELT MASKIERTEM WIRKSTOFF, DIE HERSTELLUNGSMETHODE DAZU UND ORODISPERSIBLE TABLETTEN DIESE ENTHALTEND
GRANULES COMPRISING A DOUBLE-MASKED ACTIVE AGENT, ITS METHOD OF MANUFACTURING AND ORODISPERSIBLE TABLETS CONTAINING THEM

(30) Priorité: 31.10.2014 FR 1460532
(43) Date de publication de la demande: 06.09.2017
(73) Titulaire: ETHYPHARM, 92213 Saint-Cloud Cedex (FR)
(72) Inventeur: HERRY, Catherine, F-27670 Saint-Ouen du Tilleul (FR); AILHAS, Caroline, F-27460 Alizay (FR)
(74) Mandataire: Plasseraud IP
(86) Numéro de dépôt international: PCT/FR2015/052936
(87) Numéro de publication internationale: WO 2016/066976

(56) Documents cités:
- WO-A1-2012/080408
- FR-A1- 2 848 855
- FR-A1- 2 850 275

## Description

La présente invention se rapporte au domaine de la pharmacie, et plus particulièrement à celui de la galénique.

L'invention porte sur des formulations orales, notamment des granules et comprimés orodispersibles, d'un principe actif au goût et/ou sensation en bouche particulièrement désagréables, tel que la trazodone.

### Domaine technique

Un comprimé orodispersible est une forme solide qui se désintègre ou se dissout dans la bouche, uniquement au contact de la salive, généralement en moins de 60 secondes.

Les comprimés orodispersibles représentent une forme galénique en plein essor, qui s'est beaucoup développée au cours des dernières années. En effet, les comprimés orodispersibles présentent de nombreux avantages et sont particulièrement adaptés aux patients ayant des difficultés de déglutition, par exemple les enfants et les personnes âgées. Cependant, ces populations ne sont pas les seules à présenter des problèmes de déglutition ou dysphagie, puisque environ 30 à 50 % de la population est concernée par ce problème. Sont également concernés les patients présentant des troubles psychiatriques, mais aussi ceux souffrant de troubles thyroïdiens, de la maladie de Parkinson, de maladies de déficience du système immunitaire (SIDA), de reflux gastro-intestinaux, ainsi que les patients souffrant de nausée, vomissement ou mal des transports. Les comprimés orodispersibles sont également adaptés aux personnes n'ayant pas un accès facile à l'eau, notamment lors de voyages. Un autre avantage desdits comprimés est qu'ils permettent une utilisation pratique et discrète.

Pour permettre une désintégration rapide, les comprimés orodispersibles sont de structure poreuse et sont comprimés à des pressions plus faibles que les comprimés conventionnels, les inconvénients étant qu'ils peuvent être plus fragiles et difficiles à manipuler.

Un grand nombre de méthodes pour l'obtention de comprimés orodispersibles a été mis au point durant les dernières années. Cependant il existe toujours à ce jour certaines caractéristiques limitant le développement industriel des comprimés orodispersibles, notamment leur friabilité trop importante et leur goût et sensation en bouche parfois désagréables.

Ainsi, même si les comprimés orodispersibles restent une forme assez répandue et appréciée des patients, notamment pour leur utilisation pratique et rapide, une étude effectuée par la Demanderesse a montré que le goût en bouche d'un comprimé semble être le paramètre le plus important pour les patients, et ainsi, le mauvais goût en bouche est une des causes majeures de la non-observance des traitements médicaux.

Or, de par leur nature, les comprimés orodispersibles sont destinés à se désintégrer dans la bouche, c'est-à-dire à libérer le principe actif au très mauvais goût dans la bouche. Il est donc primordial pour que le comprimé soit accepté par le patient d'avoir un masquage du goût suffisant.

Classiquement, le masquage de goût est réalisé par des techniques d'enrobage mettant en œuvre des solvants ou de l'eau. Or la règlementation pharmaceutique est de plus en plus exigeante en ce qui concerne l'absence de traces de solvants. De plus, lorsque l'enrobage est fait en milieu aqueux, une étape coûteuse de séchage est nécessaire. Par ailleurs, les quantités d'enrobage utilisables sont limitées pour assurer une libération rapide du principe actif et, souvent, insuffisantes pour obtenir un masquage de goût de qualité.

Dans les demandes de brevet FR 2 784 895 et EP 1 301 176, le masquage de goût est réalisé sans solvant par thermogranulation. Cependant, cette technique adaptée pour masquer le goût de l'ibuprofène s'avère insuffisante pour masquer le goût de principes actifs au goût beaucoup plus prononcé et désagréable, tels que la trazodone, même en augmentant considérablement les quantités d'enrobage. Par ailleurs, il s'est avéré que la technique de thermogranulation n'est pas adaptée pour mettre en œuvre des compositions binaires. De plus FR2850275, WO2012080408, FR2848855 divulguent les formes pharmaceutiques à l'administration orale qui confèrent un masquage de gout. .

Il est donc important de pouvoir disposer de granules de principe actif présentant un très mauvais goût, tel que la trazodone, qui présentent un masquage de goût satisfaisant, sans mise en œuvre de solvant, sans quantité excessive d'agents de masquage de goût et qui soient adaptés à la préparation de comprimés orodispersbles.

Ainsi, l'un des buts de l'invention est d'obtenir des granules utilisables pour un comprimé orodispersible présentant un goût agréable en bouche avec un principe actif dont le goût est reconnu comme étant particulièrement mauvais, notamment en raison de sa grande amertume, en particulier la trazodone.

Les présents inventeurs ont trouvé que ceci était possible grâce à un double masquage de goût réalisé sur le principe actif avec un composé thermofusible et un polymère thermoplastique.

### Résumé de l'invention

Ainsi, selon un premier objet, l'invention porte sur des granules de principe actif, présentant un double masquage de goût réalisé avec un composé thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges, et un polymère thermoplastique soluble à pH inférieur ou égal à 5 selon la revendication 1.

Selon un second objet, la présente invention porte sur un procédé spécifique de préparation des granules de l'invention par mise en œuvre de deux étapes successives de thermogranulation selon la revendication 5.

Selon un troisième objet, la présente invention porte sur des comprimés orodispersibles contenant les granules de l'invention selon la revendication 8.

### Description des figures :

La figure 1 représente le profil de dissolution des comprimés orodispersibles selon l'invention fabriqués selon les exemples 4 et 5.
La figure 2 représente les distances obtenues avec une langue électronique pour les comprimés fabriqués selon les exemples 3, 4 et 22.
La figure 3 représente les distances obtenues avec une langue électronique pour les comprimés fabriqués selon l'exemple 5, avec un arôme chocolat et un arôme fraise.

### Description détaillée de l'invention:

L'invention porte selon son premier objet sur un granule de principe actif présentant un très mauvais goût, tel que la trazodone, qui présente un double masquage de goût.

Dans la présente invention, on entend par principe actif, toute molécule ayant une activité thérapeutique. L'invention concerne les principes actifs ayant un très mauvais goût. Par « très mauvais goût » on entend indifféremment un goût et/ou une sensation en bouche désagréable. De tels principes actifs sont au moins aussi désagréables en bouche que l'ibuprofène. L'invention est particulièrement adaptée à la trazodone.

Dans la présente invention, par « trazodone » on entend le chlorhydrate de trazodone en tant que tel, sous forme amorphe, cristalline, sous forme base, sous forme d'hydrates ou de sels pharmaceutiquement acceptables.

Les granules de principe actif selon l'invention présentent un double masquage de goût réalisé avec un composé thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges, et un polymère thermoplastique soluble à pH inférieur ou égal à 5 selon la revendication 1.

Dans le granule à double masquage de goût, un premier masquage de goût est réalisé avec un composé thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges, et le second masquage de goût est réalisé avec un polymère thermoplastique soluble à pH inférieur ou égal à 5 selon la revendication 1.

Selon un mode de réalisation de l'invention, le composé thermofusible est choisi dans le groupe consistant en des cires, des huiles végétales hydrogénées, des acides gras, des mono-, di- et tri-esters d'acides gras et de glycérol, des triglycérides, des glycérides, des polyoxylglycérides, des alcools gras, et leurs mélanges.

Le premier masquage de goût est constitué d'un composé thermofusible, qui est solide à la température ambiante et dont la température de fusion est comprise entre 35 et 150°C. Ce composé est choisi parmi les cires, par exemple cire de carnauba, cire de candelilla, cire d'abeille, cire de paraffine ; les huiles végétales hydrogénées, par exemple les huiles de coton, de ricin ou de soja hydrogénées ; les acides gras, par exemple les acides palmitiques, stéariques et béhéniques ; les mono-, di- et tri-esters d'acides gras et de glycérol, par exemple le distéarate de glycol ; les triglycérides, par exemple la tripalmitine et la tristéarine ; les glycérides, par exemples mélange de mono-di-triglycérides ; les polyoxylglycérides, par exemple les mélanges d'esters de polyéthylène glycol, d'une fraction glycéridique et de polyéthylène glycol libre ; les alcools gras, par exemple l'alcool cétylique et l'alcool cétéarylique ; et leurs mélanges.

Ce premier masquage de goût est réalisé par thermogranulation du principe actif, en particulier de la trazodone, avec ledit composé thermofusible.

Un second masquage de goût constitué d'un polymère thermoplastique, qui est soluble au pH gastrique jusqu'à pH=5 est appliqué sur les granules comprenant le composé thermofusible.

Un tel polymère thermoplastique, soluble au pH gastrique jusqu'à pH=5, est un copolymère cationique, notamment un copolymère à base de méthacrylate d'alkyle et de méthacrylate d' alkylamine (selon la revendication 1). A titre d'exemple, on peut citer un copolymère cationique de méthylaminoéthylmétahcrylate, butylméthacrylate et méthyl méthacrylate, notamment celui commercialisé par la Société EVONIK sous la marque EUDRAGIT^{®} E PO. Tout autre EUDRAGIT^{®} E de la Société EVONIK peut être choisi comme constituant du second enrobage. Ce second masquage de goût est réalisé par thermogranulation des granules comprenant le composé thermofusible avec ledit composé thermoplastique.

Sans vouloir être liés par aucune théorie, les inventeurs sont d'avis que lors de la thermogranulation, les particules de principe actif sont engluées dans le composé thermofusible, ce dernier ne formant pas une couche homogène autour des particules de principe actif comme dans un enrobage classique, puis, lors de la seconde thermogranulation, ces particules dotées du composé thermofusible sont engluées dans le polymère thermoplastique, ce dernier ne formant pas une couche homogène autour des particules dotées du composé thermofusible comme dans un enrobage classique, mais de façon totalement inattendue, le double masquage permet de masquer efficacement le goût très désagréable du principe actif malgré la faible proportion quantitative de composé thermoactive et de composé thermofusible.

Dans la présente invention, on détermine l'efficacité du masquage de goût à l'aide d'une langue électronique Astree équipée du set #2 pour applications pharmaceutiques composé de 7 capteurs sensoriels (ZZ, AB, GA, BB, CA, DA, JE), en comparant les valeurs obtenues pour une formulation dépourvue de principe actif (placébo) avec la même formulation comprenant le principe actif. Les données générées sont traitées par analyse statistique multidimensionnelle à l'aide du logiciel AlphaSoft dans sa version V14.1. Elles permettent de définir pour chaque formulation les coordonnées d'un point et donc de calculer la distance euclidienne entre ces points. Plus cette distance sera faible meilleur sera le masquage du goût. Un masquage de goût suffisant est obtenu pour une distance euclidienne formulation-placebo de moins de 300, de préférence de moins de 260.

Le granule selon l'invention à double masquage de goût est exempt de toute trace de solvant. En effet, le granule selon l'invention est obtenu par thermogranulation. Il est donc uniquement constitué du principe actif, en particulier la trazodone, du composé thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges, et d'un polymère thermoplastique soluble à pH inférieur ou égale à 5.

Le granule de l'invention est encore caractérisé en ce que le composé thermofusible et le polymère thermoplastique sont chacun appliqués séparément et successivement par 2 thermogranulations différentes.

Selon un mode de réalisation, le granule est constitué, pour 100 parties en poids:
- de 50 à 95%, de préférence de 60 à 90% et plus préférentiellement encore de 70 à 85% en poids de principe actif, de préférence de trazodone ; et
- de 2 à 50 %, de préférence de 5 à 30% et plus préférentiellement encore de 8 à 20 % en poids de composant thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges ; et
- de 5 à 30 %, de préférence de 8 à 25% et plus préférentiellement encore de 10 à 20 % en poids de polymère thermofusible soluble à pH inférieur ou égal à 5, qui est un copolymère cationique à base de méthacrylate d'alkyle et de méthacrylate d'alkylamine selon la revendication 3.

Selon un mode de réalisation particulier, le granule selon l'invention est constitué pour 100 parties en poids:
- de 50 à 95%, de préférence de 60 à 90% et plus préférentiellement encore de 70 à 85% en poids de principe actif, de préférence de trazodone ; et
- de 10 à 25%, de préférence de 15 à 20 % en poids de distéarate de glycérol ; et
- de 5 à 20 %, de préférence de 10 à 15 % en poids de copolymère cationique de diméthylaminoéthyl méthacrylate, butyl méthacrylate et méthyl méthacrylate.

Selon un autre aspect, la présente invention porte sur un procédé de préparation des granules décrites précédemment.

Les présents inventeurs ont essayé de préparer des granules enrobés par thermogranulation d'un mélange de composé thermofusible et de polymère décrits précédemment, mais cette thermogranulation s'est avérée impossible (voir exemple comparatif 2).

Pour pallier cette difficulté, les présents inventeurs ont mis au point le procédé selon l'invention dans lequel deux étapes successives de thermogranulation sont conduites.

Ainsi, le présent procédé de préparation des granules à double masquage de goût comprend :
a) une première étape de thermogranulation, en présence du principe actif, d'un composé thermofusible choisi parmi les cires, par exemple cire de carnauba, cire de candelilla, cire d'abeille, cire de paraffine ; les huiles végétales hydrogénées, par exemple les huiles de coton, de ricin ou de soja hydrogénées ; les acides gras, par exemple les acides palmitiques, stéariques et béhéniques ; les mono-, di- et tri-esters d'acides gras et de glycérol, par exemple le distéarate de glycol ; les triglycérides, par exemple la tripalmitine et la tristéarine ; les glycérides, par exemple un mélange de mono-di-triglycérides ; les polyoxylglycérides, par exemple les mélanges d'esters de polyéthylène glycol, d'une fraction glycéridique et de polyéthylène glycol libre ; les alcools gras, par exemple l'alcool cétylique et l'alcool cétéarylique ; et leurs mélanges , ou bien d'un polymère thermoplastique soluble à pH inférieur ou égal à 5 , qui est un copolymère cationique à base de méthacrylate d'alkyle et de méthacrylate d'alkylamine selon la revendication 5; et
b) une seconde étape de thermogranulation autour du granule obtenu à l'étape a) ou bien d'un composé thermofusible choisi parmi les cires, par exemple cire de carnauba, cire de candelilla, cire d'abeille, cire de paraffine ; les huiles végétales hydrogénées, par exemple les huiles de coton, de ricin ou de soja hydrogénées ; les acides gras, par exemple les acides palmitiques, stéariques et béhéniques ; les mono-, di-et tri-esters d'acides gras et de glycérol, par exemple le distéarate de glycol ; les triglycérides, par exemple la tripalmitine et la tristéarine ; les glycérides, par exemple un mélange de mono-di-triglycérides ; les polyoxylglycérides, par exemple les mélanges d'esters de polyéthylène glycol, d'une fraction glycéridique et de polyéthylène glycol libre ; les alcools gras, par exemple l'alcool cétylique et l'alcool cétéarylique ; et leurs mélanges, ou bien d'un polymère thermoplastique soluble à pH inférieur ou égal à 5, qui est un copolymère cationique à base de méthacrylate d'alkyle et de méthacrylate d'alkylamine selon la revendication 5.

Selon un mode de réalisation particulier, le procédé comprend :
a) une première étape de thermogranulation, en présence du principe actif, de préférence de trazodone, d'un composé thermofusible choisi parmi les cires, par exemple cire de carnauba, cire de candelilla, cire d'abeille, cire de paraffine ; les huiles végétales hydrogénées, par exemple les huiles de coton, de ricin ou de soja hydrogénées ; les acides gras, par exemple les acides palmitiques, stéariques et béhéniques ; les mono-, di- et tri-esters d'acides gras et de glycérol, par exemple le distéarate de glycol ; les triglycérides, par exemple la tripalmitine et la tristéarine ; les glycérides, par exemples mélange de mono-di-triglycérides ; les polyoxylglycérides, par exemple les mélanges d'esters de polyéthylène glycol, d'une fraction glycéridique et de polyéthylène glycol libre ; les alcools gras, par exemple l'alcool cétylique et l'alcool cétéarylique ; et leurs mélanges ; et
b) une seconde étape de thermogranulation autour du granule obtenu à l'étape a) de polymère thermoplastique soluble à pH inférieur ou égale 5, , qui est un copolymère cationique à base de méthacrylate d'alkyle et de méthacrylate d'alkylamine selon les revendications 5 et 6. .

Le procédé selon l'invention est, réalisé dans un granulateur à haut cisaillement, par exemple de type Diosna P-VAC 10, qui comprend deux étapes successives de thermogranulation. Chaque étape de thermogranulation se divise en 3 phases selon le couple Température/Puissance enregistré :
- Phase de mélange : la puissance enregistrée reste stable tandis que la température du mélange augmente progressivement ;
- Phase de granulation : la température du mélange reste stable tandis que la puissance augmente ;
- Phase de refroidissement : la température du mélange et la puissance diminuent.

Les paramètres de consigne du granulateur permettant de contrôler la puissance et la température du mélange pendant le procédé sont : la vitesse de pale, la vitesse de l'émotteur et la température de consigne de la double enveloppe.

Dans la présente invention la vitesse de pale et la vitesse de l'émotteur sont exprimés en tours par minutes (tpm).

Ainsi, selon l'invention, le procédé comprend deux étapes successives de thermogranulation :
la première étape a) comprend :
le mélange de principe actif, de préférence de trazodone, et du composant thermofusible, à des vitesses constantes de pale comprises entre 500 tpm et 200 tpm et des vitesses constantes d'émotteur comprises entre 1300 tpm et 800 tpm et en augmentant la température de la matière, de la température ambiante à la température de fusion (T_{f}) du composant thermofusible +/-10°C, de préférence +/- 5°C ;
la granulation à la température de fusion (T_{f}) du composant thermofusible +/-10°C, de préférence +/- 5°C et à des vitesses constantes de pale augmentées par rapport à l'étape de mélange, comprises entre 500 tpm et 200 tpm et des vitesses constantes d'émotteur augmentées par rapport à l'étape de mélange, comprises entre 1500 tpm et 1000 tpm;
le refroidissement au cours duquel la température est abaissée jusqu'à la température ambiante et les vitesses de pale sont diminuées par rapport à celles de l'étape de granulation, comprises entre 300 tpm et 100 tpm et les vitesses d'émotteur sont diminuées par rapport à celles de l'étape de granulation, comprises entre 1500 tours par minute et 1000 tours par minute;
la seconde étape b) de thermogranulation au cours de laquelle les granules obtenus à l'étape a) sont mélangés avec le polymère soluble à pH inférieur ou égal à 5 et granulés à une température inférieure à la température mise en œuvre lors du mélange et de la granulation de l'étape a), et égale à la température de transition vitreuse (T_{g}) du composant thermoplastique +/-10°C, de préférence +/- 5°C, et à des vitesses constantes de pale diminuées par rapport à l'étape de mélange de l'étape a), comprises entre 200 tpm et 100 tpm et des vitesses constantes d'émotteur diminuées par rapport à l'étape de mélange de l'étape a), comprises entre 1000 tpm et 800 tpm, puis les granules obtenus sont refroidis jusqu'à la température ambiante avec une vitesse de pale diminuée à 100 tpm et une vitesse d'émotteur constante identique à l'étape de mélange de l'étape b).

La dimension moyenne D[4,3], mesurée par diffraction laser en voie sèche (par exemple sur un Mastersizer 2000 équipé du module Scirocco 2000) des granules finalement obtenus est de 50 µm à 500 µm, de préférence de 100 µm à 300 µm, de préférence d'environ 200µm.

La présente invention porte également sur des comprimés orodispersibles contenant les granules de l'invention ou préparés selon l'invention.

Au sens de la présente invention, un comprimé orodispersible est un comprimé qui se désintègre ou se dissout dans la bouche, uniquement au contact de la salive, sans apport d'eau et sans être mâché, en moins de 60 secondes, de préférence en moins de 40 secondes, et plus préférentiellement encore en moins de 30 secondes, en formant une suspension facile à avaler.

Le temps de désintégration (ou désagrégation) dans la bouche correspond à la durée qui sépare, d'une part, le moment de la mise en place du comprimé dans la bouche au contact de la salive et, d'autre part, le moment de la déglutition de la suspension résultant de la désintégration (désagrégation) du comprimé au contact de la salive. Ce temps de désintégration correspond au temps de désintégration *in vivo.*

On peut également mesurer le temps de désintégration *in vitro* des comprimés orodispersibles selon l'invention. Ce temps de désintégration est mesuré selon la Pharmacopée Européenne 2.9.1 sur un appareil Erweka ZT 31 ou tout autre appareil de mesure du temps de désintégration des comprimés, correspondant à la Pharmacopée Européenne 2.9.1. Le temps de désintégration *in vitro* des comprimés selon l'invention est de 10 à 30 secondes.

Le comprimé orodispersible de l'invention comprend des granules enrobés de principe actif tels que définis préalablement et un mélange d'excipients de compression choisis dans le groupe comprenant un diluant, un désintégrant, un édulcorant, un agent humectant, un lubrifiant, un agent aromatisant, un colorant et leurs mélanges. Il peut également contenir un liant et/ou un agent mouillant.

Le diluant est choisi dans le groupe comprenant le mannitol, le xylitol, le sorbitol, le maltitol et leurs mélanges.

Le désintégrant est choisi dans le groupe comprenant la crospovidone, la croscarmellose sodique (AcDiSol^{®}), le carboxyméthylamidon sodique (Explotab ^{®}) et leurs mélanges.

L'édulcorant est choisi dans le groupe comprenant l'aspartam, l'acesulfame de potassium, la saccharinate de sodium, le sucralose et leurs mélanges.

L'agent humectant est choisi dans le groupe comprenant de la silice, de préférence celle commercialisée sous la dénomination Syloïd ^{®} 244 FP, de la silice colloïdale hydrophobe, de préférence celle commercialisée sous la dénomination Aérosil ^{®} R 972, de la silice précipitée, de préférence celle commercialisée sous la dénomination Aérosil ^{®} 200, et leurs mélanges.

Le lubrifiant est choisi dans le groupe des lubrifiants hydrophobes, tel que le stéarate de magnésium ou des lubrifiants hydrophiles choisis dans le groupe comprenant le stéaryl fumarate de sodium, le lauryl sulfate de sodium.

L'agent aromatisant et le colorant pouvant entrer dans la constitution des comprimés orodispersibles sont choisis parmi ceux qui sont pharmaceutiquement acceptables. Ils sont choisis selon les caractéristiques organoleptiques souhaitées pour le comprimé orodispersible, notamment en fonction de la catégorie de patients à qui ils sont destinés. Des exemples d'agents aromatisant sont l'arôme banane, l'arôme tutti-frutti, l'arôme menthe, l'arôme fraise, l'arôme canneberge, l'arôme cassis, l'arôme caramel, l'arôme coca, l'arôme chocolat. Des arômes particulièrement adaptés sont l'arôme fraise et l'arôme chocolat.

Le liant, lorsqu'il est présent, est choisi dans le groupe comprenant l'hydroxypropylmethylcellulose, la polyvinylpyrrolidone, l'hydroxypropylcellulose faiblement substituée, la gomme arabique, l'amidon de maïs, l'amidon prégélatinisé, les maltodextrines et leurs mélanges.

L'agent mouillant, lorsqu'il est présent, est choisi dans le groupe comprenant les poloxamers, les macrogols, les macrogolglycérides, les polysorbates, ledit agent mouillant étant de préférence les macrogolglycérides commercialisés sous la dénomination Gelucire ^{®} 44/14.

Les comprimés orodispersibles selon l'invention peuvent être préparés par compression directe, c'est-à-dire par compression à sec des granules enrobés de principe actif et des excipients de compression qui sont éventuellement préalablement granulés. Selon un autre mode de réalisation, les comprimés orodispersibles selon l'invention, peuvent être préparés par compression moulage, comme décrit dans la demande de brevet FR 2 999 432 au nom de la demanderesse, auquel cas, il est possible que le mélange d'excipients comprennent en outre un agent mouillant et/ou un liant.

Selon un mode de réalisation particulier, les excipients de compression se présentent sous la forme de grains d'excipients.

Les grains d'excipients présentent une granulométrie médiane comprise entre + 30 % et - 30 %, de préférence entre + 10 % et - 10%, par rapport à la dimension des granules enrobés de principe actif. Ainsi, la dimension des grains d'excipients est de 70 µm à 650 µm, de préférence de 180 µm à 440 µm.

Selon un mode de réalisation de l'invention, le mélange d'excipients, éventuellement sous forme de grains comprend :
- de 65 à 90%, et de préférence de 70 à 80 %, d'un diluant, de préférence le mannitol commercialisé sous la dénomination Mannitol 200,
- de 2 à 25%, et de préférence de 10 à 20 %, d'un désintégrant, de préférence la crospovidone commercialisée sous la dénomination Polyplasdone ^{®} XL,
- de 1 à 8%, et de préférence de 3 à 5 %, d'un édulcorant, de préférence le sucralose,
- de 0 à 5%, et de préférence de 0,5 à 3 %, d'un agent humectant,
- de 0% à 5 %, d'un lubrifiant,
- de 0 à 8%, et de préférence de 0,5 à 4 %, d'un agent aromatisant et/ou d'un colorant,
les pourcentages étant des pourcentages en poids par rapport au poids total des comprimés.

Selon un autre mode de réalisation de l'invention, le comprimé est réalisé en appliquant des forces de compression allant de 1 à 20 kN (kNewtons), et de préférence de 2 à 6 kN.

A titre d'exemple, le comprimé orodispersible de l'invention présente :
- une masse allant de 100 à 600 mg, et de préférence de 200 à 400 mg,
- une épaisseur allant de 1 à 8 mm, et de préférence de 4 à 6 mm
- un diamètre de 7 à 14 mm, et de préférence de 8 à 12 mm.

Ainsi de tels comprimés peuvent facilement être placés dans la cavité buccale, sur la langue où ils se désintègrent du fait de la présence de la salive et de la pression naturelle exercée entre la langue et le palais lorsque la bouche est refermée.

Selon un autre mode de réalisation, les comprimés peuvent comprendre au moins une encoche permettant leur rupture afin d'administrer une quantité moindre de principe actif.

Le comprimé orodispersible selon l'invention présente une dureté allant de 20 à 80 N, et de préférence de 30 à 65 N. La méthode de mesure de la dureté est celle de la Pharmacopée Européenne 2.9.8.

A titre indicatif, le comprimé orodispersible de l'invention présente une friabilité allant de 0,0 % à 0,6 %, et de préférence de 0,1 % à 0,4 %. La friabilité est mesurée sur un appareil Erweka TA 10 selon la méthode décrite à la Pharmacopée Européenne (édition 7, chapitre 2.9.7.) En raison de cette friabilité satisfaisante, il est possible d'utiliser des méthodes industrielles classiques de transfert et de conditionnement des comprimés ne nécessitant pas de précautions particulières et permettant une grande rapidité d'exécution.

L'invention sera mieux comprise à la lumière des exemples non limitatifs et purement illustratifs suivants et des figures.

### EXEMPLES

Dans ce qui suit les produits commerciaux suivants sont utilisés:
Précirol^{®} ATO 5 : distéarate de glycérol, commercialisé par Gattefossé ;
Dynasan^{®} 114 : trimyristin, ester de glycérine substitué par des acides gras en C14, commercialisé par Cremer Oleo Division ;
Softisan^{®} 154 : dérivé d'huile de palme hydrogénée, mélange de triglycérides (palmitique et stéarique) et d'acides gras ayant une longueur de chaines allant de C10 à C18, commercialisé par Cremer Oleo Division ;
Lipoxol^{®} 3 350: polyéthylènes Glycols, connus sous des noms commerciaux tels que Macrogols, PEG ou encore Carbowax, commercialisé par Sasol ;
Compritol^{®} HD5 ATO: Behenoyl Polyoxyl-8 glycerides, mélange de PEG et de Mono, di et triglycérides, commercialisé par Gattefossé ;
Montane^{®} 60 PHA: stéarate de sorbitan, dérivé de sorbitol et d'acide stéarique, commercialisé par Seppic ;
Crodacol^{®} CS 50: alcool cétostéarylique, mélange de chaînes à 16 atomes de carbone et de chaînes à 18 atomes de carbone, commercialisé par Croda Inc ;
Crodacol^{®} S 95 : alcool stéarylique, alcool gras avec une longueur de chaine de 18 atomes de carbone, commercialisé par Croda Inc ;
Crodacol^{®} C 95 : alcool cétylique, alcool gras avec une longueur de chaine de 16 atomes de carbone, commercialisé par Croda Inc ;
Bitrex : Benzoate de Denatonium, substance la plus amère découverte à ce jour, utilisé comme agent amérisant afin de simuler l'amertume d'un principe actif ;
Eudragit^{®} E PO : copolymère cationique à base de diméthylaminoéthyl méthacrylate, butyl méthacrylate et méthyl méthacrylate- (numéro CAS 24938 - 16 - 7) commercialisé par Evonik ;
AcDiSol^{®} : croscarmellose sodique, commercialisée par FMC Biopolymer ;
Polyplasdone XL : crospovidone, commercialisée par ISP Pharamaceutical ;
Mannitol SD 200: mannitol, commercialisé par Roquette ; Syloid^{®} 244FP : Silice, commercialisée par Grace Davison.

### Exemple 1 :

### Etape 1 : Thermogranulation à l'aide de Précirol ATO 5

Le principe actif et le Précirol ATO 5 sont introduits dans un granulateur à haut cisaillement de type Diosna P-VAC10 dans les proportions indiquées dans le Tableau 1. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

A titre informatif, les pourcentages donné dans la présente invention sont un rapport masse par masse (m/m).

**Tableau 1**

| Formule centésimale du grain | | |
|---|---|---|
| Ingrédients | | % (m/m) |
| Trazodone HCl | Principe Actif | 80,00 |
| Precirol ATO 5 | Liant thermofusible | 20,00 |
| *Total* | | *100,0* |

Selon l'Exemple 1, les paramètres de consignes utilisés, à savoir: la vitesse de pale, la vitesse de l'émotteur et la température de consigne de la double enveloppe, sont indiqués dans le Tableau 2.

**Tableau 2.**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 500 | 1000 | 65 | 400 | 1500 | 57 | 200 à 100 | 1000 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Précirol ATO 5 et d'Eudragit E PO

Le grain obtenu selon l'étape 1 ci-dessus est introduit dans un granulateur haut cisaillement de type Diosna P-VAC10 dans les proportions indiquées dans le Tableau 3. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 3**

| Formule centésimale du grain | | | |
|---|---|---|---|
| Ingrédients | | | % (m/m) |
| Grain exemple 1 | Trazodone HCl | Principe Actif | 72,00 |
| | Precirol ATO 5 | Liant thermofusible | 18,00 |
| Eudragit E PO | | Polymère thermoplastique | 10,00 |
| *Total* | | | 100,0 |

Les paramètres de consignes utilisés sont indiqués dans le Tableau 4.

**Tableau 4**

| Mélange | | | Granulation | | | Refroidissement | | |
|---|---|---|---|---|---|---|---|---|
| V pale (tpm) | V émotteur (tpm) | T double enveloppe (°C) | V pale (tpm) | V émotteur (tpm) | T double enveloppe (°C) | V pale (tpm) | V émotteur (tpm) | T double enveloppe (°C) |
| 250 | 1000 | 48 | 250 | 1000 | 52 | 200 à 100 | 1000 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 2 (comparatif) : Thermogranulation à l'aide d'un mélange Précirol ATO 5 / Eudragit E PO

Le principe actif, le Précirol ATO 5 et l'Eudragit E PO sont introduits dans un granulateur haut cisaillement de type Diosna P-VAC10 dans les proportions indiquées dans le Tableau 5. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 5**

| Formule centésimale du grain | | |
|---|---|---|
| Ingrédients | | % (m/m) |
| Trazodone HCl | Principe Actif | 72,00 |
| Precirol ATO 5 | Liant thermofusible | 18,00 |
| Eudragit E PO | Polymère thermoplastique | 10,00 |
| *Total* | | 100,0 |

Selon l'exemple 2, les paramètres de consignes utilisés sont indiqués dans le Tableau 6.

**Tableau 6**

| Mélange | | | Granulation | | | Refroidissement | | |
|---|---|---|---|---|---|---|---|---|
| V pale (tpm) | V émotteur (tpm) | T double enveloppe (°C) | V pale (tpm) | V émotteur (tpm) | T double enveloppe (°C) | V pale (tpm) | V émotteur (tpm) | T double enveloppe (°C) |
| 300 | 1000 | 65 | 300 | 1500 | 60 | 300-100 | 1000 | 10 |

La thermogranulation à l'aide d'un mélange Précirol ATO 5 et Eudragit E PO n'est techniquement pas contrôlable : une surgranulation à l'étape de la granulation engendre une surgranulation majeure au refroidissement. Le grain obtenu se présente alors sous la forme d'une pâte dure pouvant entraîner un endommagement du granulateur haut cisaillement et rendant son calibrage impossible dans des rendements compatibles avec un procédé industriel.

### Exemple 3 : Compression du grain de l'étape 1 de l'exemple 1, dosage 30mg

Le grain fabriqué à l'issue de l'étape 1 de l'Exemple 1 est mis en œuvre dans la fabrication de comprimés, dosés à 30mg en chlorhydrate de Trazodone. Le mélange puis la lubrification sont effectués selon les proportions indiquées dans le Tableau 3 à l'aide d'un mélangeur cubique équipé d'une cuve de taille adaptée ou de tout autre équipement assurant une bonne homogénéité de mélange.

**Tableau 3**

| composition centésimale comprimé orodispersible | | | | Dosage 30mg |
|---|---|---|---|---|
| Ingrédients | | | %(m/m) | mg / comprimé |
| Grain Eexemple 1 | Trazodone HCl API | Principe Actif | 15,00 | 30,00 |
| | Precirol ATO 5 | Liant thermofusible | 3,75 | 7,50 |
| AcDisol | | Désintégrant | 5,00 | 10,00 |
| Polyplasdone XL | | Désintégrant | 2,00 | 4,00 |
| Mannitol SD 200 | | Diluant | 68,45 | 136,90 |
| Arome | | Arome | 1,00 | 2,00 |
| Sucralose | | Edulcorant | 1,00 | 2,00 |
| Colorant | | Colorant | 1,00 | 2,00 |
| Syloid 244FP | | Humectant | 2,00 | 4,00 |
| Stéarate de Mg | | Lubrifiant | 0,80 | 1, 60 |
| *Total* | | | 100,0 | 200,0 |

Le mélange est comprimé sur presse rotative Fette 102i équipée d'un distributeur gravitaire et de 3 jeux de matrice/poinçons ronds, diamètre 8 mm, une barre de sécabilité. La masse unitaire cible est de 200 mg et la force de compression ajustée pour obtenir une dureté cible de 60 N.

### Exemple 4 : Compression du grain de l'Exemple 1, dosage 30mg

Le grain fabriqué selon l'Exemple 1 est mis en œuvre dans la fabrication de comprimés orodispersibles, dosés à 30mg en chlorhydrate de Trazodone. Le mélange puis la lubrification sont effectués selon les proportions indiquées dans le Tableau 4 à l'aide d'un mélangeur cubique équipé d'une cuve de taille adaptée ou de tout autre équipement assurant une bonne homogénéité de mélange.

**Tableau 4**

| composition centésimale comprimé orodispersible | | | | Dosage 30mg |
|---|---|---|---|---|
| Ingrédients | | | % (m/m) | mg / comprimé |
| Grain etape 2 | Trazodone HCl API | Principe Actif | 15,00 | 30,00 |
| | Precirol ATO 5 | Liant thermofusible | 3,75 | 7,50 |
| | Eudragit E PO | Polymère thermoplastique | 2,08 | 4,17 |
| AcDisol | | Désintégrant | 5,00 | 10,00 |
| Polyplasdone XL | | Désintégrant | 2,00 | 4,00 |
| Mannitol SD 200 | | Diluant | 66,37 | 132,73 |
| Arome | | Arome | 1,00 | 2,00 |
| Sucralose | | Edulcorant | 1,00 | 2,00 |
| Colorant | | Colorant | 1,00 | 2,00 |
| Syloid 244FP | | Humectant | 2,00 | 4,00 |
| Stéarate de Mg | | Lubrifiant | 0,80 | 1, 60 |
| *Total* | | | 100,0 | 200,0 |

Le mélange est comprimé sur presse rotative Fette 102i équipée d'un distributeur gravitaire et de 3 jeux de matrice/poinçons ronds, diamètre 8mm, 1 barre de sécabilité. La masse unitaire cible est de 200mg et la force de compression ajustée pour obtenir une dureté cible de 35N.

Selon l'Exemple 4, l'arôme utilisé peut être un arôme fraise ou chocolat (arômes fournis par Firmenich, grade pharmaceutique).

### Exemple 5 : Compression du grain de l'Exemple 1, dosage 90mg

Le grain fabriqué selon l'Exemple 1 est mis en œuvre dans la fabrication de comprimés orodispersibles, dosés à 90mg en Chlorhydrate de Trazodone. Le mélange puis la lubrification sont effectués selon les proportions indiquées dans le Tableau 5 à l'aide d'un mélangeur cubique équipé d'une cuve de taille adaptée ou de tout autre équipement assurant une bonne homogénéité de mélange.

**Tableau 5**

| Formule centésimale | | | | Dosage 90mg |
|---|---|---|---|---|
| Ingrédients | | | % (m/m) | mg / comprimé |
| Grain Exemple 3 | Trazodone HCl API | Principe Actif | 22,50 | 90,00 |
| | Precirol ATO 5 | Liant thermofusible | 5,63 | 22,50 |
| | Eudragit E PO | Polymère thermoplastique | 3,13 | 12,50 |
| AcDisol | | Désintégrant | 5,00 | 20,00 |
| Polyplasdone XL | | Désintégrant | 2,00 | 8,00 |
| Mannitol SD 200 | | Diluant | 55,95 | 223,80 |
| Arome | | Arome | 1,00 | 4,00 |
| Sucralose | | Edulcorant | 1,00 | 4,00 |
| Colorant | | Colorant | 1,00 | 4,00 |
| Syloid 244FP | | Humectant | 2,00 | 8,00 |
| Stéarate de Mg | | Lubrifiant | 0,80 | 3,20 |
| *Total* | | | 100,0 | 400,0 |

Le mélange est comprimé sur presse rotative Fette 102i équipée d'un distributeur gravitaire et de 3 jeux de matrice/poinçons ronds, diamètre 11mm. La masse unitaire cible est de 400mg et la force de compression ajustée pour obtenir une dureté cible de 30N.

### Exemple 6 :

### Etape 1 : Thermogranulation à l'aide de Dynasan 114 + Trazodone

Le principe actif et le Dynasan 114 sont introduits dans un granulateur à haut cisaillement de type Diosna P-VAC10 dans les proportions indiquées dans le Tableau 10. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 10**

| Formule centésimale du grain | | | |
|---|---|---|---|
| Ingredients | | % (m/m) | Poids (grammes) |
| Trazodone HCL | Principe actif | 77,05 | 402,9 |
| Dynasan 114 | Liant thermofusible | 22,9 | 120,0 |
| Total | | 100,0 | 522,9 |

Les paramètres de consignes utilisés, à savoir: la vitesse de pale, la vitesse de l'émotteur et la température de consigne de la double enveloppe, sont indiqués dans le Tableau 11.

**Tableau 11**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 300 | 1000 | 70 | 300 | 1500 | 65 | 100 | 1000 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Dynasan 114 et d'Eudragit E PO

Le grain obtenu à l'étape 1 est introduit dans un granulateur haut cisaillement de type Diosna P-VAC10 dans les proportions indiquées dans le Tableau 12. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 12**

| Formule centésimale du grain | | | |
|---|---|---|---|
| Ingrédients | | | % (m/m) |
| Grain Exemple 7 | Trazodone HCl | Principe Actif | 90,00 |
| | Dyanasan 114 | Liant thermofusible | |
| Eudragit E PO | | Polymère thermoplastique | 10,00 |
| *Total* | | | 100,0 |

Les paramètres de consignes utilisés sont indiqués dans le Tableau 13.

**Tableau 13**

| Mélange | | | Refroidissement | | |
|---|---|---|---|---|---|
| V pale (tpm) | V émotteur (tpm) | T double enveloppe (°C) | V pale (tpm) | V émotteur (tpm) | T double enveloppe (°C) |
| 300 | 800 | 53 | 100 | 800 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 7 : Compression du grain de l'Exemple 6, dosage 60mg

Le grain fabriqué selon l'Exemple 6 est mis en œuvre dans la fabrication de comprimés, dosés à 60mg en chlorhydrate de Trazodone. Le mélange puis la lubrification sont effectués selon les proportions indiquées dans le tableau 14 à l'aide d'un mélangeur cubique équipé d'une cuve de taille adaptée ou de tout autre équipement assurant une bonne homogénéité de mélange.

**Tableau 14**

| composition centésimale comprimé orodispersible | | | | Dosage 60mg |
|---|---|---|---|---|
| Ingrédients | | | % (m/m) | mg / comprimé |
| Grain Exemple 8 | Trazodone HCl | Principe Actif | 20,00 | 60,00 |
| | Dynasan 114 | Liant thermofusible | 5,00 | 15,00 |
| | Eudragit E PO | Polymère thermoplastique | 2,94 | 8,83 |
| AcDisol | | Désintégrant | 2,00 | 6,00 |
| Crospovidone XL | | Désintégrant | 5,00 | 15,00 |
| Mannitol SD 200 | | Diluant | 60,26 | 180,77 |
| Arome | | Arome | 1,00 | 3,00 |
| Sucralose | | Edulcorant | 1,00 | 3,00 |
| Syloid 244FP | | Humectant | 2,00 | 6,00 |
| Stéarate de Mg | | Lubrifiant | 0,80 | 2,40 |
| *Total* | | | 100,0 | 300,0 |

Le mélange est comprimé sur presse rotative Fette 102i équipée d'un distributeur gravitaire et de 3 jeux de matrice/poinçons ronds bombé, diamètre 10 mm, une barre de sécabilité. La masse unitaire cible est de 300 mg et la force de compression ajustée pour obtenir une dureté cible de 22 N.

### Exemple 8 :

### Etape 1 : Thermogranulation à l'aide de Softisan 154 + Trazodone

Le principe actif et le Softisan 154 sont introduits dans un granulateur à haut cisaillement de type Diosna P-VAC10 dans les proportions indiquées dans le Tableau 15. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 15**

| Formule centésimale du grain | | | |
|---|---|---|---|
| Ingredients | | % (m/m) | Poids (grammes) |
| Trazodone HCL | Principe actif | 80 | 480,00 |
| Softisan 154 | Liant thermofusible | 20 | 120,00 |
| Total | | 100,0 | 600,00 |

Les paramètres de consignes utilisés, à savoir: la vitesse de pale, la vitesse de l'émotteur et la température de consigne de la double enveloppe, sont indiqués dans le Tableau 16.

**Tableau 16.**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 300 | 1000 | 65 | 300 | 1500 | 63-65 | 100 | 1000 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Softisan 154 et d'Eudragit E PO

Le grain obtenu à l'étape 1 est introduit dans un granulateur haut cisaillement de type Diosna P-VAC10 dans les proportions indiquées dans le Tableau 17. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 17**

| Formule centésimale du grain | | | |
|---|---|---|---|
| Ingrédients | | | % (m/m) |
| Grain Exemple 10 | Trazodone HCl | Principe Actif | 90,00 |
| | Softisan 154 | Liant thermofusible | |
| Eudragit E PO | | Polymère thermoplastique | 10,00 |
| *Total* | | | 100,0 |

Les paramètres de consignes utilisés sont indiqués dans le tableau 18.

**Tableau 18**

| Mélange | | | Refroidissement | | |
|---|---|---|---|---|---|
| V pale (tpm) | V émotteur (tpm) | T double enveloppe (°C) | V pale (tpm) | V émotteur (tpm) | T double enveloppe (°C) |
| 300 | 800 | 53 | 100 | 800 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 9 : Compression du grain de l'Exemple 8, dosage 60mg

Le grain fabriqué selon l'Exemple 8 est mis en œuvre dans la fabrication de comprimés, dosés à 60mg en chlorhydrate de Trazodone. Le mélange puis la lubrification sont effectuées selon les proportions indiquées dans le Tableau 19 à l'aide d'un mélangeur cubique équipé d'une cuve de taille adaptée ou de tout autre équipement assurant une bonne homogénéité de mélange.

**Tableau 19**

| composition centésimale comprimé orodispersible | | | | Dosage 60mg |
|---|---|---|---|---|
| Ingrédients | | | % (m/m) | mg / comprimé |
| Grain Exemple 11 | Trazodone HCl | Principe Actif | 20,00 | 60,00 |
| | Dynasan 114 | Liant thermofusible | 5,00 | 15,00 |
| | Eudragit E PO | Polymère thermoplastique | 2,94 | 8,83 |
| AcDisol | | Désintégrant | 2,00 | 6,00 |
| Crospovidone XL | | Désintégrant | 5,00 | 15,00 |
| Mannitol SD 200 | | Diluant | 60,26 | 180,77 |
| Arome | | Arome | 1,00 | 3,00 |
| Sucralose | | Edulcorant | 1,00 | 3,00 |
| Syloid 244FP | | Humectant | 2,00 | 6,00 |
| Stéarate de Mg | | Lubrifiant | 0,80 | 2,40 |
| *Total* | | | 100,0 | 300,0 |

Le mélange est comprimé sur presse rotative Fette 102i équipée d'un distributeur gravitaire et de 3 jeux de matrice/poinçons ronds bombé, diamètre 10 mm, une barre de sécabilité. La masse unitaire cible est de 300 mg et la force de compression ajustée pour obtenir une dureté cible de 25 N.

### Exemple 10 :

### Etape 1 : Thermogranulation à l'aide de Lipoxol 3350 + Trazodone

Le principe actif et le Lipoxol 3350 sont introduits dans un granulateur à haut cisaillement de type Diosna P-VAC10 dans les proportions indiquées dans le Tableau 20. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 20**

| Formule centésimale du grain | | | |
|---|---|---|---|
| Ingredients | | % (m/m) | Poids (grammes) |
| Trazodone HCL | Principe actif | 80 | 480,00 |
| Lipoxol 3350 | Liant thermofusible | 20 | 120,00 |
| Total | | 100,0 | 600,00 |

Les paramètres de consignes utilisés, à savoir: la vitesse de pale, la vitesse de l'émotteur et la température de consigne de la double enveloppe, sont indiqués dans le Tableau 21.

**Tableau 21**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 300 | 1000 | 70 | 300 | 1500 | 65 | 100 | 1000 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Lipoxol 3350 et d'Eudragit E PO

Le grain obtenu à l'étape 1 est introduit dans un granulateur haut cisaillement de type Diosna P-VAC10 dans les proportions indiquées dans le Tableau 22. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 22**

| Formule centésimale du grain | | | |
|---|---|---|---|
| Ingrédients | | | % (m/m) |
| Grain Exemple 13 | Trazodone HCl | Principe Actif | 90,00 |
| | Lipoxol 3350 | Liant thermofusible | |
| Eudragit E PO | | Polymère thermoplastique | 10,00 |
| *Total* | | | 100,0 |

Les paramètres de consignes utilisés sont indiqués dans le tableau 23.

**Tableau 23**

| Mélange | | | Refroidissement | | |
|---|---|---|---|---|---|
| V pale (tpm) | V émotteur (tpm) | T double enveloppe (°C) | V pale (tpm) | V émotteur (tpm) | T double enveloppe (°C) |
| 200 | 800-700 | 53 | 100 | 700 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 11 : Compression du grain de l'Exemple 10, dosage 60mg

Le grain fabriqué selon l'Exemple 10 est mis en œuvre dans la fabrication de comprimés, dosés à 60mg en chlorhydrate de Trazodone. Le mélange puis la lubrification sont effectuées selon les proportions indiquées dans le Tableau 24 à l'aide d'un mélangeur cubique équipé d'une cuve de taille adaptée ou de tout autre équipement assurant une bonne homogénéité de mélange.

**Tableau 24**

| composition centésimale comprimé orodispersible | | | | Dosage 60mg |
|---|---|---|---|---|
| Ingrédients | | | % (m/m) | mg / comprimé |
| Grain Exemple 14 | Trazodone HCl | Principe Actif | 20,00 | 60,00 |
| | Lipoxol 3350 | Liant thermofusible | 5,00 | 15,00 |
| | Eudragit E PO | Polymère thermoplastique | 2,94 | 8,83 |
| AcDisol | | Désintégrant | 2,00 | 6,00 |
| Crospovidone XL | | Désintégrant | 5,00 | 15,00 |
| Mannitol SD 200 | | Diluant | 60,26 | 180,77 |
| Arome | | Arome | 1,00 | 3,00 |
| Sucralose | | Edulcorant | 1,00 | 3,00 |
| Syloid 244FP | | Humectant | 2,00 | 6,00 |
| Stéarate de Mg | | Lubrifiant | 0,80 | 2,40 |
| *Total* | | | 100,0 | 300,0 |

Le mélange est comprimé sur presse rotative Fette 102i équipée d'un distributeur gravitaire et de 3 jeux de matrice/poinçons ronds bombé, diamètre 10 mm, une barre de sécabilité. La masse unitaire cible est de 300 mg et la force de compression ajustée pour obtenir une dureté cible de 18 N.

### Exemple 12 :

### Etape 1 : Thermogranulation à l'aide de Compritol HD5 ATO + Trazodone

Le principe actif et le Compritol HD5 ATO sont introduits dans un granulateur à haut cisaillement de type Diosna P-VAC10 dans les proportions indiquées dans le Tableau 25. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 25**

| Formule centésimale du grain | | | |
|---|---|---|---|
| Ingredients | | % (m/m) | Poids (grammes) |
| Trazodone HCL | Principe actif | 80 | 480,00 |
| Compritol HD5 ATO | Liant thermofusible | 20 | 120,00 |
| Total | | 100,0 | 600,00 |

Les paramètres de consignes utilisés, à savoir: la vitesse de pale, la vitesse de l'émotteur et la température de consigne de la double enveloppe, sont indiqués dans le Tableau 26.

**Tableau 26**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 300 | 1000 | 70 | 300 | 1500 | 68-65 | 100 | 1000 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Compritol HD5 ATO et d'Eudragit E PO

Le grain obtenu à l'étape 1 est introduit dans un granulateur haut cisaillement de type Diosna P-VAC10 dans les proportions indiquées dans le Tableau 27. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 27**

| Formule centésimale du grain | | | |
|---|---|---|---|
| Ingrédients | | | % (m/m) |
| Grain Exemple 16 | Trazodone HCl | Principe Actif | 90,00 |
| | Compritol HD5 ATO | Liant thermofusible | |
| Eudragit E PO | | Polymère thermoplastique | 10,00 |
| *Total* | | | 100,0 |

Les paramètres de consignes utilisés sont indiqués dans le tableau 28.

**Tableau 28**

| Mélange | | | Refroidissement | | |
|---|---|---|---|---|---|
| V pale (tpm) | V émotteur (tpm) | T double enveloppe (°C) | V pale (tpm) | V émotteur (tpm) | T double enveloppe (°C) |
| 200-300 | 800 | 53 | 100 | 800 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 13 : Compression du grain de l'exemple 12, dosage 60mg

Le grain fabriqué selon l'Exemple 12 est mis en œuvre dans la fabrication de comprimés, dosés à 60mg en chlorhydrate de Trazodone. Le mélange puis la lubrification sont effectuées selon les proportions indiquées dans le Tableau 29 à l'aide d'un mélangeur cubique équipé d'une cuve de taille adaptée ou de tout autre équipement assurant une bonne homogénéité de mélange.

**Tableau 29**

| composition centésimale comprimé orodispersible | | | | Dosage 60mg |
|---|---|---|---|---|
| Ingrédients | | | %(m/m) | mg / comprimé |
| Grain Exemple 17 | Trazodone HCl | Principe Actif | 20,00 | 60,00 |
| | Compritol HD5 ATO | Liant thermofusible | 5,00 | 15,00 |
| | Eudragit E PO | Polymère thermoplastique | 2,94 | 8,83 |
| AcDisol | | Désintégrant | 2,00 | 6,00 |
| Crospovidone XL | | Désintégrant | 5,00 | 15,00 |
| Mannitol SD 200 | | Diluant | 60,26 | 180,77 |
| Arome | | Arome | 1,00 | 3,00 |
| Sucralose | | Edulcorant | 1,00 | 3,00 |
| Syloid 244FP | | Humectant | 2,00 | 6,00 |
| Stéarate de Mg | | Lubrifiant | 0,80 | 2,40 |
| *Total* | | | 100,0 | 300,0 |

Le mélange est comprimé sur presse rotative Fette 102i équipée d'un distributeur gravitaire et de 3 jeux de matrice/poinçons ronds bombé, diamètre 10 mm, une barre de sécabilité. La masse unitaire cible est de 300 mg et la force de compression ajustée pour obtenir une dureté cible de 27 N.

### Exemple 14 :

### Etape 1 : Thermogranulation à l'aide de Montane 60 PHA + Bitrex

Un mélange de Bitrex et de Lactose 200M représentant le principe actif ainsi que le Montane 60 PHA sont introduits dans un granulateur à haut cisaillement de type Diosna P-VAC10 dans les proportions indiquées dans le Tableau 30. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 30**

| Formule centésimale du grain | | | |
|---|---|---|---|
| Ingredients | | % (m/m) | Poids (grammes) |
| Bitrex | Agent amer | 80 | 0,25 |
| Lactose 200M | Diluant | 79,95 | 399,75 |
| Montane 60PHA | Liant thermofusible | 20 | 100,00 |
| Total | | 100,0 | 500,00 |

Les paramètres de consignes utilisés, à savoir: la vitesse de pale, la vitesse de l'émotteur et la température de consigne de la double enveloppe, sont indiqués dans le Tableau 31.

**Tableau 31**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 300 | 1000 | 70 | 300 | 1500 | 60 | 100 | 1000-1500 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Montane 60 PHA et d'Eudragit E PO

Le grain obtenu à l'étape 1 est introduit dans un granulateur haut cisaillement de type Diosna P-VAC10 dans les proportions indiquées dans le Tableau 32. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 32**

| Formule centésimale du grain | | | |
|---|---|---|---|
| Ingrédients | | | % (m/m) |
| Grain Exemple 19 | Bitrex | Agent amer | 90,00 |
| | Lactose 200M | Diluant | |
| | Montane 60PHA | Liant thermofusible | |
| Eudragit E PO | | Polymère thermoplastique | 10,00 |
| *Total* | | | 100,0 |

Les paramètres de consignes utilisés sont indiqués dans le tableau 33.

**Tableau 33**

| Mélange | | | Refroidissement | | |
|---|---|---|---|---|---|
| V pale (tpm) | V émotteur (tpm) | T double enveloppe (°C) | V pale (tpm) | V émotteur (tpm) | T double enveloppe (°C) |
| 150 | 800 | 53 | 100 | 800 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 15 : Compression du grain de l'Exemple 14, dosage 60mg

Le grain fabriqué selon l'exemple 14 est mis en œuvre dans la fabrication de comprimés dosés à 60mg d'un mélange de Lactose 200M et de Bitrex. Le mélange puis la lubrification sont effectués selon les proportions indiquées dans le Tableau 34 à l'aide d'un mélangeur cubique équipé d'une cuve de taille adaptée ou de tout autre équipement assurant une bonne homogénéité de mélange.

**Tableau 34**

| composition centésimale comprimé orodispersible | | | | Dosage 60mg |
|---|---|---|---|---|
| Ingrédients | | | % (m/m) | mg / comprimé |
| | Bitrex | Agent amer | 0,0125 | 0,0375 |
| Grain Exemple 20 | Lactose 200M | Diluant | 19,98 | 59,94 |
| | Montane 60PHA | Liant thermofusible | 5,00 | 14,99 |
| | Eudragit E PO | Polymère thermoplastique | 2,78 | 8,33 |
| AcDisol | | Désintégrant | 2,00 | 6,00 |
| Crospovidone XL | | Désintégrant | 5,00 | 15,00 |
| Mannitol SD 200 | | Diluant | 60,43 | 181,30 |
| Arome | | Arome | 1,00 | 3,00 |
| Sucralose | | Edulcorant | 1,00 | 3,00 |
| Syloid 244FP | | Humectant | 2,00 | 6,00 |
| Stéarate de Mg | | Lubrifiant | 0,80 | 2,40 |
| *Total* | | | 100,0 | 300,0 |

Le mélange est comprimé sur presse rotative Fette 102i équipée d'un distributeur gravitaire et de 3 jeux de matrice/poinçons ronds bombé, diamètre 10 mm, une barre de sécabilité. La masse unitaire cible est de 300 mg et la force de compression ajustée pour obtenir une dureté cible de 18 N.

### Exemple 16 :

### Etape 1 : Thermogranulation à l'aide de Crodacol CS 50 + Bitrex

Un mélange de Bitrex et de Lactose 200M représentant le principe actif ainsi que le Crodacol CS 50 sont introduits dans un granulateur à haut cisaillement de type Diosna P-VAC10 dans les proportions indiquées dans le Tableau 35. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 35**

| Formule centésimale du grain | | | |
|---|---|---|---|
| Ingredients | | % (m/m) | Poids (grammes) |
| Bitrex | Agent amer | 80 | 0,25 |
| Lactose 200M | Diluant | 79,95 | 399,75 |
| Crodacol CS 50 | Liant thermofusible | 20 | 100,00 |
| Total | | 100,0 | 500,00 |

Les paramètres de consignes utilisés, à savoir: la vitesse de pale, la vitesse de l'émotteur et la température de consigne de la double enveloppe, sont indiqués dans le Tableau 36.

**Tableau 36**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 300 | 1000 | 70 | 300 | 1500 | 65 | 100-200 | 1000-1500 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Crodacol CS 50 et d'Eudragit E PO

Le grain obtenu selon l'étape 1 est introduit dans un granulateur haut cisaillement de type Diosna P-VAC10 dans les proportions indiquées dans le Tableau 37. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 37**

| Formule centésimale du grain | | | |
|---|---|---|---|
| Ingrédients | | | %(m/m) |
| Grain Exemple 22 | Bitrex | Agent amer | 90,00 |
| | Lactose 200M | Diluant | |
| | Crodacol CS 50 | Liant thermofusible | |
| Eudragit E PO | | Polymère thermoplastique | 10,00 |
| *Total* | | | 100,0 |

Les paramètres de consignes utilisés sont indiqués dans le tableau 38.

**Tableau 38**

| Mélange | | | Refroidissement | | |
|---|---|---|---|---|---|
| V pale (tpm) | V émotteur (tpm) | T double enveloppe (°C) | V pale (tpm) | V émotteur (tpm) | T double enveloppe (°C) |
| 100 | 800 | 53 | 100 | 800 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 17 : Compression du grain de l'Exemple 16, dosage 60mg

Le grain fabriqué selon l'exemple 16 est mis en œuvre dans la fabrication de comprimés dosés à 60mg d'un mélange de Lactose 200M et de Bitrex. Le mélange puis la lubrification sont effectuées selon les proportions indiquées dans le Tableau 39 à l'aide d'un mélangeur cubique équipé d'une cuve de taille adaptée ou de tout autre équipement assurant une bonne homogénéité de mélange.

**Tableau 39**

| composition centésimale comprimé orodispersible | | | | Dosage 60mg |
|---|---|---|---|---|
| Ingrédients | | | % (m/m) | mg / comprimé |
| Grain Exemple 23 | Bitrex | Agent amer | 0,0125 | 0,0375 |
| | Lactose 200M | Diluant | 19,98 | 59,94 |
| | Crodacol CS 50 | Liant thermofusible | 5,00 | 14,99 |
| | Eudragit E PO | Polymère thermoplastique | 2,78 | 8,33 |
| AcDisol | | Désintégrant | 2,00 | 6,00 |
| Crospovidone XL | | Désintégrant | 5,00 | 15,00 |
| Mannitol SD 200 | | Diluant | 60,43 | 181,30 |
| Arome | | Arome | 1,00 | 3,00 |
| Sucralose | | Edulcorant | 1,00 | 3,00 |
| Syloid 244FP | | Humectant | 2,00 | 6,00 |
| Stéarate de Mg | | Lubrifiant | 0,80 | 2,40 |
| *Total* | | | 100,0 | 300,0 |

Le mélange est comprimé sur presse rotative Fette 102i équipée d'un distributeur gravitaire et de 3 jeux de matrice/poinçons ronds bombé, diamètre 10 mm, une barre de sécabilité. La masse unitaire cible est de 300 mg et la force de compression ajustée pour obtenir une dureté cible de 19 N.

### Exemple 18 :

### Etape 1 : Thermogranulation à l'aide de Crodacol S 95 + Bitrex

Un mélange de Bitrex et de Lactose 200M représentant le principe actif ainsi que le Crodacol S 95 sont introduits dans un granulateur à haut cisaillement de type Diosna P-VAC10 dans les proportions indiquées dans le Tableau 40. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 40**

| Formule centésimale du grain | | | |
|---|---|---|---|
| Ingredients | | % (m/m) | Poids (grammes) |
| Bitrex | Agent amer | 80 | 0,25 |
| Lactose 200M | Diluant | 79,95 | 399,75 |
| Crodacol S 95 | Liant thermofusible | 20 | 100,00 |
| Total | | 100,0 | 500,00 |

Les paramètres de consignes utilisés, à savoir: la vitesse de pale, la vitesse de l'émotteur et la température de consigne de la double enveloppe, sont indiqués dans le Tableau 41.

**Tableau 41**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 300 | 1000 | 70 | 300 | 1500 | 65 | 100 | 1000-1500 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Crodacol S 95 et d'Eudragit E PO

Le grain à l'étape 1 est introduit dans un granulateur haut cisaillement de type Diosna P-VAC10 dans les proportions indiquées dans le Tableau 42. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 42**

| Formule centésimale du grain | | | |
|---|---|---|---|
| Ingrédients | | | % (m/m) |
| Grain Exemple 25 | Bitrex | Agent amer | 90,00 |
| | Lactose 200M | Diluant | |
| | Crodacol S 95 | Liant thermofusible | |
| Eudragit E PO | | Polymère thermoplastique | 10,00 |
| *Total* | | | 100,0 |

Les paramètres de consignes utilisés sont indiqués dans le tableau 43.

**Tableau 43**

| Mélange | | | Refroidissement | | |
|---|---|---|---|---|---|
| V pale (tpm) | V émotteur (tpm) | T double enveloppe (°C) | V pale (tpm) | V émotteur (tpm) | T double enveloppe (°C) |
| 150 | 800 | 55 | 100 | 800 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 19 : Compression du grain de l'Exemple 18, dosage 60mg

Le grain fabriqué selon l'exemple 18 est mis en œuvre dans la fabrication de comprimés dosés à 60mg d'un mélange de Lactose 200M et de Bitrex. Le mélange puis la lubrification sont effectuées selon les proportions indiquées dans le Tableau 44 à l'aide d'un mélangeur cubique équipé d'une cuve de taille adaptée ou de tout autre équipement assurant une bonne homogénéité de mélange.

**Tableau 44**

| composition centésimale comprimé orodispersible | | | | Dosage 60mg |
|---|---|---|---|---|
| Ingrédients | | | % (m/m) | mg / comprimé |
| Grain Exemple 26 | Bitrex | Agent amer | 0,0125 | 0,0375 |
| | Lactose 200M | Diluant | 19,98 | 59,94 |
| | Crodacol S 95 | Liant thermofusible | 5,00 | 14,99 |
| | Eudragit E PO | Polymère thermoplastique | 2,78 | 8,33 |
| AcDisol | | Désintégrant | 2,00 | 6,00 |
| Crospovidone XL | | Désintégrant | 5,00 | 15,00 |
| Mannitol SD 200 | | Diluant | 60,43 | 181,30 |
| Arome | | Arome | 1,00 | 3,00 |
| Sucralose | | Edulcorant | 1,00 | 3,00 |
| Syloid 244FP | | Humectant | 2,00 | 6,00 |
| Stéarate de Mg | | Lubrifiant | 0,80 | 2,40 |
| *Total* | | | 100,0 | 300,0 |

Le mélange est comprimé sur presse rotative Fette 102i équipée d'un distributeur gravitaire et de 3 jeux de matrice/poinçons ronds bombé, diamètre 10 mm, une barre de sécabilité. La masse unitaire cible est de 300 mg et la force de compression ajustée pour obtenir une dureté cible de 16 N.

### Exemple 20 :

### Etape 1 : Thermogranulation à l'aide de Crodacol C 95 + Bitrex

Un mélange de Bitrex et de Lactose 200M représentant le principe actif ainsi que le Crodacol C 95 sont introduits dans un granulateur à haut cisaillement de type Diosna P-VAC10 dans les proportions indiquées dans le Tableau 45. En fonction de la densité du mélange, la masse totale introduite est adaptée par l'homme de l'art au volume de remplissage de la cuve du granulateur de façon à garantir son homogénéisation tout au long du procédé de thermogranulation (idéalement 2/3 du volume).

**Tableau 45**

| Formule centésimale du grain | | | |
|---|---|---|---|
| Ingredients | | % (m/m) | Poids (grammes) |
| Bitrex | Agent amer | 80 | 0,25 |
| Lactose 200M | Diluant | 79,95 | 399,75 |
| Crodacol S 95 | Liant thermofusible | 20 | 100,00 |
| Total | | 100,0 | 500,00 |

Les paramètres de consignes utilisés, à savoir: la vitesse de pale, la vitesse de l'émotteur et la température de consigne de la double enveloppe, sont indiqués dans le Tableau 46.

**Tableau 46**

| Mélange | | | Granulation | | | Refroidissement | | |
|---|---|---|---|---|---|---|---|---|
| V pale (tpm) | V émotteur (tpm) | T double enveloppe (°C) | V pale (rpm) | V émotteur (tpm) | T double enveloppe (°C) | V pale (tpm) | V émotteur (tpm) | T double enveloppe (°C) |
| 300 | 1000 | 65 | 300 | 1500 | 60 | 100 | 1000 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10cpm.

### Etape 2 : Thermogranulation à l'aide de Crodacol C 95 et d'Eudragit E PO

Le grain obtenu à l'étape 1 est introduit dans un granulateur haut cisaillement de type Diosna P-VAC10 dans les proportions indiquées dans le Tableau 47. En fonction de la densité du mélange, la masse totale introduite est adaptée au volume de remplissage de la cuve du granulateur de façon à garantir son homogénéisation tout au long du procédé de thermogranulation (idéalement 2/3 du volume).

**Tableau 47**

| Formule centésimale du grain | | | |
|---|---|---|---|
| Ingrédients | | | % (m/m) |
| Grain Exemple 28 | Bitrex | Agent amer | 90,00 |
| | Lactose 200M | Diluant | |
| | Crodacol C 95 | Liant thermofusible | |
| Eudragit E PO | | Polymère thermoplastique | 10,00 |
| *Total* | | | *100,0* |

Les paramètres de consignes utilisés sont indiqués dans le tableau 48.

**Tableau 48**

| Mélange | | | Refroidissement | | |
|---|---|---|---|---|---|
| V pale (tpm) | V émotteur (tpm) | T double enveloppe (°C) | V pale (tpm) | V émotteur (tpm) | T double enveloppe (°C) |
| 200 | 800 | 53 | 100 | 800 | 10 |

Une fois revenu à température ambiante, le grain obtenu est déchargé et calibré à l'aide d'un bâti-moteur ERWEKA AR402 équipé d'un calibreur oscillant type FGS sur grille de 500µm, à une vitesse de 10 cpm.

### Exemple 21 : Compression du grain de l'Exemple 20, dosage 60mg

Le grain fabriqué selon l'exemple 20 est mis en œuvre dans la fabrication de comprimés, dosés à 60mg d'un mélange de Lactose 200M et de Bitrex. Le mélange puis la lubrification sont effectuées selon les proportions indiquées dans le tableau 49 à l'aide d'un mélangeur cubique équipé d'une cuve de taille adaptée ou de tout autre équipement assurant une bonne homogénéité de mélange.

**Tableau 49**

| composition centésimale comprimé orodispersible | | | | Dosage 60mg |
|---|---|---|---|---|
| Ingrédients | | | % (m/m) | mg / comprimé |
| Grain Exemple 29 | Bitrex | Agent amer | 0,0125 | 0,0375 |
| | Lactose 200M | Diluant | 19,98 | 59,94 |
| | Crodacol C 95 | Liant thermofusible | 5,00 | 14,99 |
| | Eudragit E PO | Polymère thermoplastique | 2,78 | 8,33 |
| AcDisol | | Désintégrant | 2,00 | 6,00 |
| Crospovidone XL | | Désintégrant | 5,00 | 15,00 |
| Mannitol SD 200 | | Diluant | 60,43 | 181,30 |
| Arome | | Arome | 1,00 | 3,00 |
| Sucralose | | Edulcorant | 1,00 | 3,00 |
| Syloid 244FP | | Humectant | 2,00 | 6,00 |
| Stéarate de Mg | | Lubrifiant | 0,80 | 2,40 |
| *Total* | | | 100,0 | 300,0 |

Le mélange est comprimé sur presse rotative Fette 102i équipée d'un distributeur gravitaire et de 3 jeux de matrice/poinçons ronds bombé, diamètre 10 mm, une barre de sécabilité. La masse unitaire cible est de 300 mg et la force de compression ajustée pour obtenir une dureté cible de 14 N.

### Exemple 22: Compression du principe actif sans masquage de goût

Le principe actif est mis en œuvre dans la fabrication de comprimés, dosés à 30 mg en chlorhydrate de Trazodone. Le mélange puis la lubrification sont effectuées selon les proportions indiquées dans le Tableau 50 à l'aide d'un mélangeur cubique équipé d'une cuve de taille adaptée ou de tout autre équipement assurant une bonne homogénéité de mélange.

**Tableau 50**

| Formule centésimale | | | Dosage 30mg |
|---|---|---|---|
| Ingrédients | | % (m/m) | mg / comprimé |
| Trazodone HCl API | Principe Actif | 15,00 | 30,00 |
| AcDisol | Désintégrant | 5,00 | 10,00 |
| Polyplasdone XL | Désintégrant | 2.00 | 4,00 |
| Mannitol SD 200 | Diluant | 72,20 | 144,40 |
| Arome | Arome | 1,00 | 2,00 |
| Sucralose | Edulcorant | 1,00 | 2,00 |
| Colorant | Colorant | 1,00 | 2,00 |
| Syloid 244FP | Humectant | 2,00 | 4,00 |
| Stéarate de Mg | Lubrifiant | 0,80 | 1, 60 |
| *Total* | | 100,0 | 200,0 |

Le mélange est comprimé sur presse rotative Fette 102i équipée d'un distributeur gravitaire et de 3 jeux de matrice/poinçons ronds, diamètre 8 mm, 1 barre de sécabilité. La masse unitaire cible est de 200 mg et la force de compression ajustée pour obtenir une dureté cible de 60 N.

### Exemple 23 : Caractérisation physique des comprimés

### Mesure de la masse des comprimés

La masse des comprimés est mesurée sur une balance de type Mettler Toledo AG245 (précision 0,1/0,01mg) sur un échantillon représentatif de 10 comprimés.

### Mesure de la dureté

La dureté est mesurée selon la méthode décrite à la Pharmacopée Européenne 8.0 (chapitre 2.9.8. « Résistance à la rupture des comprimés »).

### Mesure de la friabilité

La friabilité est mesurée selon la méthode décrite à la Pharmacopée Européenne 8.0 (chapitre 2.9.7. « Friabilité des comprimés non enrobés »).

### Mesure du temps de désintégration

Le temps de désintégration est mesuré selon la méthode décrite à la Pharmacopée Européenne 8.0 (chapitre 2.9.1. « Friabilité des comprimés non enrobés »).

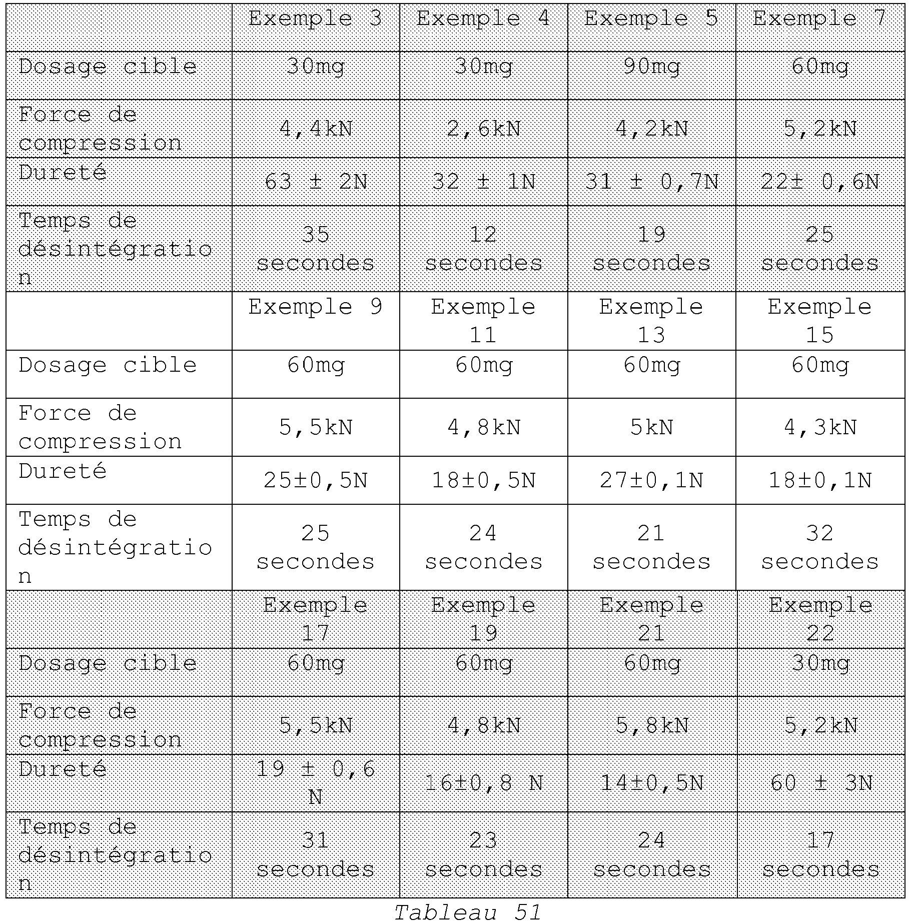

### Exemple 24 : Mesure de la dissolution du principe actif

La dissolution du chlorhydrate de Trazodone est mesurée sous agitation continue après introduction des comprimés dans un appareil de dissolution de type 2 décrit à la Pharmacopée Européenne 8.0 (chapitre 2.9.3. « Essai de dissolution des formes solides) équipé de palettes (vitesse d'agitation 50 tpm). Le milieu de dissolution utilisé est constitué de 500mL d'HCl 0,01N et est maintenu à une température constante de 37°C (± 0,5° C). Le dosage continu du principe actif est mesuré par détection UV à λ=311 nm (cuves 10 mm).

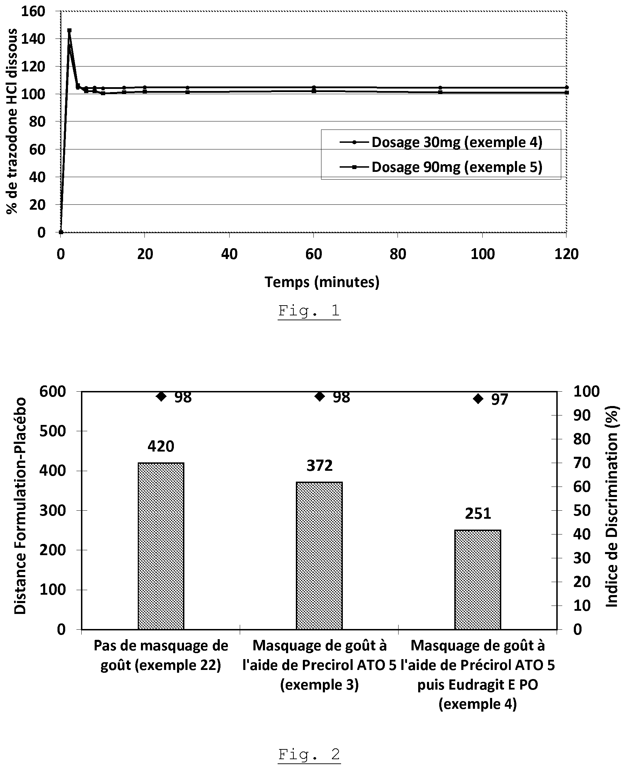

Les résultats obtenus pour les comprimés fabriqués selon les exemples 5 et 6 sont reportés sur la Figure 1.

### Exemple 25 : Mesure du masquage de goût par langue électronique

La mesure du masquage de goût est réalisée à l'aide d'une langue électronique Astree équipée du set #2 pour applications pharmaceutiques composé de 7 capteurs sensoriels ((ZZ, AB, GA, BB, CA, DA, JE).

Chaque comprimé est dissous dans de l'eau déionisée (2 comprimés dans 50mL). Après désintégration complète sous agitation magnétique, les suspensions sont filtrées sur papier (porosité de 10 à 20µm). Les solutions filtrées sont versées dans des béchers de 25mL et placées sur un passeur automatique de type carrousel à 48 positions pour analyse immédiate.

Les conditions d'analyses sont les suivantes :
volume d'échantillon de 25 mL,
Temps d'acquisition de 120 secondes,
Durée d'analyse de 180 secondes.

Le signal mesuré par la langue électronique est mesuré à l'équilibre (obtenu en 100 à 120 secondes en moyenne) sur les 7 capteurs. La mesure est prise trois fois pour chaque échantillon et les capteurs sont rincés à l'eau déionisée entre chaque mesure.

Ces mesures sont réalisées sur les comprimés des exemples 3, 4 et 22.

Des comprimés placebo sont préparés de la même façon que les comprimés des exemples 3, 4 et 22 mais sans principe actif. La mesure par langue électronique est également réalisée trois fois à l'aide de ces comprimés placébo.

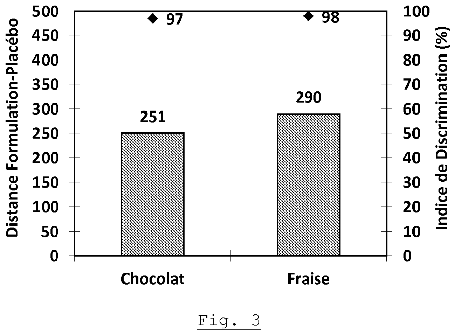

Les données générées sont traitées par analyse statistique multidimensionnelle à l'aide du logiciel AlphaSoft dans sa version V14.1. Pour chaque couple comprimé-placebo, le logiciel calcule la distance euclidienne entre les valeurs obtenues avec la formulation contenant le principe actif et les valeurs obtenues avec le placébo correspondant. Plus cette valeur est faible, plus le masquage du goût de la Trazodone HCl est efficace. Les résultats obtenus pour les exemples 3, 4 et 22 sont reportés sur les Figures 2 et 3.

## Revendications

1. Granule de principe actif à double masquage de goût, dans lequel le double masquage de goût est réalisé par :
- un composé thermofusible choisi parmi les cires, les huiles végétales hydrogénées, les acides gras, les mono-, diet tri-esters d'acides gras et de glycérol, les triglycérides, les glycérides, les polyoxylglycérides, les alcools gras, et leurs mélanges, et
- un polymère thermoplastique soluble à pH inférieur ou égal à 5, qui est un copolymère cationique à base de méthacrylate d'alkyle et de méthacrylate d'alkylamine,
et dans lequel le composé thermofusible et le polymère thermoplastique sont chacun appliqués séparément par thermogranulation.

2. Granule selon la revendication 1, **caractérisé en ce que** le granule à double masquage de goût est exempt de toute trace de solvant.

3. Granule selon la revendication 1 ou 2, **caractérisé par le fait qu'**il est constitué, pour 100 parties en poids
- de 50 à 95%, de préférence de 60 à 90% et plus préférentiellement encore de 70 à 85% en poids de principe actif, de préférence de trazodone;
- de 2 à 50 %, de préférence de 5 à 30% et plus préférentiellement encore de 8 à 20 % en poids de composant thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges ; et
- de 5 à 30 %, de préférence de 8 à 25% et plus préférentiellement encore de 10 à 20 % en poids de polymère thermoplastique soluble à pH inférieur ou égal à 5 qui est un copolymère cationique à base de méthacrylate d'alkyle et de méthacrylate d'alkylamine.

4. Granule selon la revendication 3, qui est constitué pour 100 parties en poids:
- de 50 à 95%, de préférence de 60 à 90% et plus préférentiellement encore de 70 à 85% en poids de principe actif, de préférence de trazodone ;
- de 10 à 25%, de préférence de 15 à 20 % en poids de distéarate de glycérol ; et
- de 5 à 20 %, de préférence de 10 à 15 % en poids de copolymère cationique de diméthylaminoéthyl méthacrylate, butyl méthacrylate et méthyl méthacrylate.

5. Procédé de préparation de granules de trazodone tels que définis à l'une quelconque des revendications 1 à 4, **caractérisé par le fait qu'**il comprend :
a) une première étape de thermogranulation, en présence de principe actif, de préférence de trazodone, ou bien d'un composé thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges ; ou bien d'un polymère thermoplastique soluble à pH inférieur ou égale à 5 qui est un copolymère cationique à base de méthacrylate d'alkyle et de méthacrylate d'alkylamine ; et
b) une seconde étape de thermogranulation autour du granule obtenu à l'étape a) ou bien d'un composé thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges ; ou bien d'un polymère thermoplastique soluble à pH inférieur ou égale à 5 qui est un copolymère cationique à base de méthacrylate d'alkyle et de méthacrylate d'alkylamine.

6. Procédé selon la revendication 5, comprenant:
a) une première étape de thermogranulation, en présence de principe actif, de préférence de trazodone, d'un composé thermofusible choisi parmi les cires, les huiles végétales hydrogénées, les acides gras, les mono-, di- et tri-esters d'acides gras et de glycérol, les triglycérides, les glycérides, les polyoxylglycérides, les alcools gras, et leurs mélanges ; et
b) une seconde étape de thermogranulation autour du granule obtenu à l'étape a) d'un polymère thermoplastique soluble à pH inférieur ou égale à 5 qui est un copolymère cationique à base de méthacrylate d'alkyle et de méthacrylate d'alkylamine.

7. Procédé selon la revendication 6, dans lequel
la première étape a) comprend le mélange de principe actif, de préférence de trazodone et du composé thermofusible à des vitesses de pale et d'émotteur constantes en augmentant la température de la matière, de la température ambiante à la température de fusion (Tf) du composé thermofusible +/-10°C, de préférence +/- 5°C; la granulation à la température de fusion (Tf) du composé thermofusible +/-10°C, de préférence +/- 5°C et à des vitesses de pale et d'émotteur augmentées par rapport à l'étape de mélange ; le refroidissement au cours duquel la température est abaissée jusqu'à la température ambiante et les vitesses de pale et d'émotteur sont diminuées par rapport à celles de l'étape de mélange ;
la seconde étape b) de thermogranulation comprend le mélange des granules obtenus à l'étape a) avec le polymère thermoplastique soluble à pH inférieur ou égal à 5 qui est un copolymère cationique à base de méthacrylate d'alkyle et de méthacrylate d'alkylamine, la granulation à une température inférieure à la température mise en œuvre lors du mélange et de la granulation de l'étape a), et égale à la température de transition vitreuse (T_{g}) du polymère thermoplastique +/-10°C , de préférence +/- 5°C, et à des vitesses de pale et d'émotteur diminuées par rapport à l'étape de mélange de l'étape a), puis le refroidissement des granules obtenus jusqu'à la température ambiante avec une vitesse de pale diminuée.

8. Comprimé orodispersible comprenant des granules enrobés de principe actif tels que définis à l'une quelconque des revendications 1 à 4 ou tels qu'obtenus par le procédé de l'une quelconque des revendications 5 à 7, et un mélange d'excipients de compression choisis dans le groupe comprenant un diluant, un désintégrant, un édulcorant, un agent humectant, un lubrifiant, un agent aromatisant, un colorant et leurs mélanges, et éventuellement un liant et/ou un agent mouillant.

9. Comprimé orodispersible selon la revendication 8, **caractérisé par le fait que** les excipients de compression se présentent sous la forme de grains d'excipients.

## Patentansprüche

1. Wirkstoffgranulat mit doppelter Geschmacksmaskierung, wobei die doppelte Geschmacksmaskierung erreicht wird durch:
- eine Heißschmelzverbindung, ausgewählt aus Wachsen, hydrierten Pflanzenölen, Fettsäuren, Mono-, Di- und Triestern von Fettsäuren und Glycerin, Triglyceriden, Glyceriden, Polyoxylglyceriden, Fettalkoholen und deren Mischungen, und
- ein thermoplastisches Polymer, das bei einem pH-Wert von 5 oder darunter löslich ist, bei dem es sich um ein kationisches Copolymer auf der Basis von Alkylmethacylat und Alkylaminmethacrylat handelt,
und wobei die Heißschmelzverbindung und das thermoplastische Polymer jeweils getrennt durch Thermogranulierung aufgebracht werden.

2. Granulat nach Anspruch 1, **dadurch gekennzeichnet, dass** das Granulat mit doppelter Geschmacksmaskierung frei von jeglichen Spuren von Lösungsmitteln ist.

3. Granulat nach Anspruch 1 oder 2, **dadurch gekennzeichnet, dass** es, bezogen auf 100 Gewichtsteile, aus den folgenden Bestandteilen zusammengesetzt ist:
- 50 bis 95 Gew.-%, vorzugsweise 60 bis 90 Gew.-% und noch bevorzugter 70 bis 85 Gew.-% des Wirkstoffs, vorzugsweise Trazodon;
- 2 bis 50 Gew.-%, vorzugsweise 5 bis 30 Gew.-% und noch bevorzugter 8 bis 20 Gew.-% einer Heißschmelzkomponente, ausgewählt aus Wachsen, hydrierten Pflanzenölen, Fettsäuren, Mono-, Di- und Triestern von Fettsäuren und Glycerin, Triglyceriden, Glyceriden, Polyoxylglyceriden, Fettalkoholen und deren Mischungen; und
- 5 bis 30 Gew.-%, vorzugsweise 8 bis 25 Gew.-% und noch bevorzugter 10 bis 20 Gew.-% eines thermoplastischen Polymers, das bei einem pH-Wert von 5 oder darunter löslich ist, bei dem es sich um ein kationisches Copolymer auf der Basis von Alkylmethacrylat und Alkylaminmethacrylat handelt.

4. Granulat nach Anspruch 3, welches, bezogen auf 100 Gewichtsteile, aus folgenden Bestandteilen zusammengesetzt ist:
- 50 bis 95 Gew.-%, vorzugsweise 60 bis 90 Gew.-% und noch bevorzugter 70 bis 85 Gew.-% eines Wirkstoffs, vorzugsweise Trazodon;
- 10 bis 25 Gew.-%, vorzugsweise 15 bis 20 Gew.-% Glycerindistearat; und
- 5 bis 20 Gew.-%, vorzugsweise von 10 bis 15 Gew.-%, kationisches Copolymer aus Dimethylaminoethylmethacrylat, Butylmethacrylat und Methylmethacrylat.

5. Verfahren zur Herstellung von Trazodongranulat, wie in einem der Ansprüche 1 bis 4 definiert, **dadurch gekennzeichnet, dass** es umfasst:
a) einen ersten Schritt der Thermogranulierung in Gegenwart des Wirkstoffs, vorzugsweise Trazodon, entweder einer Heißschmelzverbindung, ausgewählt aus Wachsen, hydrierten Pflanzenölen, Fettsäuren, Mono-, Di- und Triestern von Fettsäuren und Glycerin, Triglyceriden, Glyceriden, Polyoxylglyceriden, Fettalkoholen und deren Mischungen; oder eines thermoplastischen Polymers, das bei einem pH-Wert von 5 oder darunter löslich ist, bei dem es sich um ein kationisches Copolymer auf der Basis von Alkylmethacylat und Alkylaminmethacrylat handelt; und
b) einen zweiten Schritt der Thermogranulierung um das in Schritt a) erhaltene Granulat herum, entweder einer Heißschmelzverbindung, ausgewählt aus Wachsen, hydrierten Pflanzenölen, Fettsäuren, Mono-, Di- und Triestern von Fettsäuren und Glycerin, Triglyceriden, Glyceriden, Polyoxylglyceriden, Fettalkoholen und deren Mischungen; oder eines thermoplastischen Polymers, das bei einem pH-Wert von 5 oder darunter löslich ist und bei dem es sich um ein kationisches Copolymer auf der Basis von Alkylmethacrylat und Alkylaminmethacrylat handelt.

6. Verfahren nach Anspruch 5, umfassend:
a) einen ersten Schritt der Thermogranulierung, in Gegenwart des Wirkstoffs, vorzugsweise Trazodon, einer Heißschmelzverbindung, ausgewählt aus Wachsen, hydrierten Pflanzenölen, Fettsäuren, Mono-, Di- und Triestern von Fettsäuren und Glycerin, Triglyceriden, Glyceriden, Polyoxylglyceriden, Fettalkoholen und deren Mischungen; und
b) einen zweiten Schritt der Thermogranulierung um das in Schritt a) erhaltene Granulat herum, eines thermoplastischen Polymers, das bei einem bei pH-Wert von 5 oder darunter löslich ist, bei dem es sich um ein kationisches Coploymer auf der Basis von Alkylmethacrylat und Alkylaminmethacrylat handelt.

7. Verfahren nach Anspruch 6, wobei
der erste Schritt a) umfasst das Mischen des Wirkstoffs, vorzugsweise Trazodon, und der Heißschmelzverbindung bei konstanten Schaufel- und Krümelgeschwindigkeiten unter Erhöhung der Temperatur des Materials von Raumtemperatur auf die Schmelztemperatur (Tf) der Heißschmelzverbindung +/-10°C, vorzugsweise +/- 5°C; Granulieren bei der Schmelztemperatur (Tf) der Heißschmelzverbindung +/-10°C, vorzugsweise +/- 5°C, und bei im Vergleich zum Mischschritt erhöhten Schaufel- und Krümelgeschwindigkeiten; und Abkühlen, wobei die Temperatur auf Raumtemperatur gesenkt wird und die Schaufel- und Krümelgeschwindigkeiten im Vergleich zum Mischschritt verringert werden;
der zweite Schritt b) der Thermogranulierung umfasst das Mischen des in Schritt a) erhaltenen Granulats mit dem thermoplastischen Polymer, das bei einem pH-Wert von 5 oder darunter löslich ist, bei dem es sich um ein kationisches Copolymer auf der Basis von Acrylmethacrylat und Alkylaminmethacrylat handelt, die Granulierung bei einer Temperatur, die niedriger ist als die beim Mischen und Granulieren in Schritt a) eingesetzte Temperatur, und gleich der Glasübergangstemperatur (T_{g}) des thermoplastischen Polymers +/-10°C , vorzugsweise +/- 5°C ist, und bei verminderten Schaufel- und Krümelgeschwindigkeiten im Vergleich zum Mischschritt von Schritt a), und anschließendes Abkühlen der erhaltenen Granulate auf Raumtemperatur mit verminderter Schaufelgeschwindigkeit.

8. Orodispersible Tablette, umfassend beschichtete Wirkstoffgranulate, wie in einem der Ansprüche 1 bis 4 definiert oder wie durch das Verfahren nach einem der Ansprüche 5 bis 7 erhalten, und eine Mischung von Tablettierhilfsstoffen, ausgewählt aus der Gruppe bestehend aus einem Verdünnungsmittel, einem Sprengmittel, einem Süßstoff, einem Feuchthaltemittel, einem Gleitmittel, einem Aromastoff, einem Farbstoff und Mischungen davon, und gegebenenfalls einem Bindemittel und/oder einem Netzmittel.

9. Orodispersible Tablette nach Anspruch 8, **dadurch gekennzeichnet, dass** die Tablettierhilfsstoffe in Form von Hilfsstoffkörnern vorliegen.

## Claims

1. A granule of active ingredient with double taste masking, wherein the double taste masking is achieved by:
- a hot-melt compound selected from waxes, hydrogenated vegetable oils, fatty acids, mono-, di- and triesters of fatty acids and of glycerol, triglycerides, glycerides, polyoxylglycerides, fatty alcohols, and mixtures thereof, and
- a thermoplastic polymer that is soluble at a pH less than or equal to 5, which is a cationic copolymer based on alkyl methacrylate and on alkylamine methacrylate,
and wherein the hot-melt compound and the thermoplastic polymer are each applied separately by means of thermal granulation.

2. The granule as claimed in claim 1, **characterized in that** the granule with double taste masking is free of any trace of solvent.

3. The granule as claimed in claim 1 or 2, **characterized in that** it consists, for 100 parts by weight:
- of from 50% to 95%, preferably from 60% to 90% and even more preferentially from 70% to 85% by weight of active ingredient, preferably trazodone;
- of from 2% to 50%, preferably from 5% to 30% and even more preferentially from 8% to 20% by weight of hot-melt component selected from waxes, hydrogenated vegetable oils, fatty acids, mono-, di- and triesters of fatty acids and of glycerol, triglycerides, glycerides, polyoxylglycerides, fatty alcohols, and mixtures thereof; and
- of from 5% to 30%, preferably from 8% to 25% and even more preferentially from 10% to 20% by weight of thermoplastic polymer that is soluble at a pH less than or equal to 5 which is a cationic copolymer based on alkyl methacrylate and on alkylamine methacrylate.

4. The granule as claimed in claim 3, which consists, for 100 parts by weight:
- of from 50% to 95%, preferably from 60% to 90% and even more preferentially from 70% to 85% by weight of active ingredient, preferably trazodone;
- of from 10% to 25%, preferably from 15% to 20% by weight of glyceryl distearate; and
- of from 5% to 20%, preferably from 10% to 15% by weight of cationic copolymer of dimethylaminoethyl methacrylate, butyl methacrylate and methyl methacrylate.

5. A method for producing trazodone granules as defined in any one of claims 1 to 4, **characterized in that** it comprises:
a) a first step of thermal granulation, in the presence of active ingredient, preferably of trazodone, or else of a hot-melt compound selected from waxes, hydrogenated vegetable oils, fatty acids, mono-, di- and triesters of fatty acids and of glycerol, triglycerides, glycerides, polyoxylglycerides, fatty acids, and mixtures thereof; or else of a thermoplastic polymer that is soluble at a pH less than or equal to 5 which is a cationic copolymer based on alkyl methacrylate and on alkylamine methacrylate; and
b) a second step of thermal granulation around the granule obtained in step a) or else a hot-melt compound selected from waxes, hydrogenated vegetable oils, fatty acids, mono-, di- and triesters of fatty acids and of glycerol, triglycerides, glycerides, polyoxylglycerides, fatty alcohols, and mixtures thereof; or else a thermoplastic polymer that is soluble at a pH less than or equal to 5 which is a cationic copolymer based on alkyl methacrylate and on alkylamine methacrylate.

6. The method as claimed in claim 5, comprising:
a) a first step of thermal granulation, in the presence of active ingredient, preferably of trazodone, of a hot-melt compound selected from waxes, hydrogenated vegetable oils, fatty acids, mono-, diand triesters of fatty acids and of glycerol, triglycerides, glycerides, polyoxylglycerides, fatty alcohols, and mixtures thereof; and
b) a second step of thermal granulation around the granule obtained in step a) of a thermoplastic polymer that is soluble at a pH less than or equal to 5 which is a cationic copolymer based on alkyl methacrylate and on alkylamine methacrylate.

7. The method as claimed in claim 6, wherein
the first step a) comprises mixing of active ingredient, preferably of trazodone, and of the hot-melt compound at constant paddle and lump breaker speeds while increasing the temperature of the material, from ambient temperature to the melting point (Mp) of the hot-melt compound +/-10°C, preferably +/- 5°C; granulation at the melting point (Mp) of the hot-melt compound +/-10°C, preferably +/- 5°C and at paddle and lump breaker speeds that are increased relative to the mixing step; cooling during which the temperature is decreased to ambient temperature and the paddle and lump breaker speeds are decreased relative to those of the mixing step;
the second step b) of thermal granulation comprises mixing of the granules obtained in step a) with the thermoplastic polymer that is soluble at a pH less than or equal to 5 which is a cationic copolymer based on alkyl methacrylate and on alkylamine methacrylate, granulation at a temperature below the temperature used during the mixing and the granulation of step a), and equal to the glass transition temperature (T_{g}) of the thermoplastic polymer +/-10°C, preferably +/- 5°C, and at paddle and lump breaker speeds that are decreased relative to the mixing step of step a), then cooling of the granules obtained to ambient temperature with a decreased paddle speed.

8. An orodispersible tablet comprising coated granules of active ingredient as defined in any one of claims 1 to 4 or as obtained by means of the method of claim 5 to 7, and a mixture of compression excipients selected from the group comprising a diluent, a disintegrant, a sweetener, a humectant, a lubricant, a flavoring agent, a dye and mixtures thereof, and optionally a binder and/or a wetting agent.

9. The orodispersible tablet as claimed in claim 8, **characterized in that** the compression excipients are in the form of grains of excipients.
